# EUROPEAN PATENT SPECIFICATION

(11) **EP 4 185 339 B1**
(45) Date of publication and mention of the grant of the patent: **01.01.2025**
(21) Application number: 21751841.4
(22) Date of filing: 19.07.2021
(51) Int. Cl.: A61L 2/28, A61B 90/70, G06V 20/52, G06V 40/20, A61L 2/18

(54) **VALIDATION OF PROCEDURES IN A DECONTAMINATION PROCESS**
VALIDIERUNG VON VERFAHREN IN EINEM DEKONTAMINATIONSVERFAHREN
VALIDATION DE PROCÉDURES DANS UN PROCESSUS DE DÉCONTAMINATION

(30) Priority: 20.07.2020 GB 202011194
(43) Date of publication of application: 31.05.2023
(73) Proprietor: Tristel PLC, Snailwell, Newmarket CB8 7NY (GB)
(72) Inventor: BRAND, Thomas, Cambridgeshire CB8 7NY (GB); JANSEN, Esther, Cambridgeshire CB8 7NY (GB); SWINNEY, Paul, Cambridgeshire CB8 7NY (GB)
(74) Representative: Dummett Copp LLP
(86) International application number: PCT/GB2021/051850
(87) International publication number: WO 2022/018421

(56) References cited:
- CN-A- 110 263 689
- US-A1- 2020 043 604
- US-B2- 9 433 474

## Description

### BACKGROUND

### a. Field of the Invention

The present invention relates to methods and apparatus for validating procedural steps during use of a decontamination system. The invention is particularly for use in validating processes for disinfecting medical devices and surfaces in clinical environments.

### b. Related Art

Effective decontamination of medical devices, surfaces and other objects in clinical environments is essential to ensure patient safety. Several highly-effective disinfectant systems have been developed to address this need.

For example, WO 2005/011756 discloses a two-part disinfectant system. The disinfectant system comprises a first part having a first reagent in a carrier medium and a second part which is miscible with the first part and which comprises a second reagent in a carrier medium. The first reagent and the second reagent react when mixed to provide a disinfecting composition. In a preferred implementation, one of the parts is an acidic solution and the other of the parts is a solution containing sodium chlorite or sodium chlorate, and a disinfecting composition comprising chlorine dioxide is produced when the two parts are mixed. The first part, known as an activator, is contained in a pump dispenser whereby it may be dispensed as a fluid, preferably as a foam, and the second part is absorbed or impregnated in at least one fabric member in a sealed container. To prepare a disinfecting wipe, a user removes an impregnated wipe from the container, and applies a portion of foam from the dispenser to the wipe. To facilitate mixing of the reagents in the foam and the wipe, the user may fold the wipe to surround the foam and crush or rub the folded wipe by scrunching before opening it out.

WO 2005/107823 describes a decontamination system (shown in Figure 1) suitable for the reprocessing of non-lumened medical devices using a manual three-wipe disinfection process. An example system includes a box 10 containing sachets 11 of pre-clean wipes, a disinfecting system 12 including a dispenser 14 and sachets 16 of disinfecting wipes as discussed above, and a box 18 containing sachets 20 of sterile rinse wipes. The pre clean wipe is used to wipe an item such as an endoscope which is to be decontaminated. The two-part disinfecting system 12 (combination of a wipe and activator foam) is used for sterilising or disinfecting the item and the sterile rinse wipe is used to remove any chemical residue. All disinfection details can be recorded in an accompanying audit trail book to allow full procedural traceability.

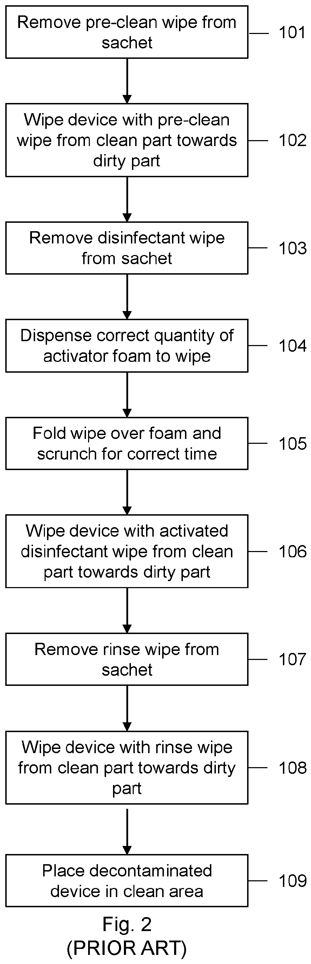

To ensure that a decontamination system of this type is fully effective, it is necessary for the user to follow correctly a specific sequence of steps. An example of such a sequence is shown in Figure 2.

In step 101, a pre-clean wipe is removed from its sachet 11. In step 102, the device is wiped with the pre-clean wipe, starting at the cleanest part of the device (such as a handle) and wiping towards the dirtiest or most contaminated part (such as an invasive distal part).

In step 103, a disinfectant wipe is removed from its sachet 16 and opened out; then in step 104 the correct quantity of activator foam is dispensed onto the wipe (typically measured by operating the dispenser 14 a predefined number of times). In step 105, the wipe is folded to enclose the foam and then scrunched for a predefined period of time to thoroughly mix the two parts of the disinfectant system. In step 106, the device is wiped with the activated disinfectant wipe, again starting at the cleanest part and moving towards the dirtiest part.

In step 107, a rinse wipe is removed from the sachet 20, and in step 108 the device is wiped with the rinse wipe, again in the clean-to-dirty direction.

In step 109, the decontaminated device is placed into a designated clean area, to avoid re-contamination of the device that might otherwise occur.

Any errors made by the user can compromise the decontamination process. Examples of such errors include selection of the wrong sachet 11, 16, 20 in any of steps 101, 103 or 107, wiping in the wrong direction or too quickly in any of steps 102, 106 or 108, dispensing an insufficient amount of activator in step 104, and scrunching the wipe for too short a time in step 105.

The use of an audit trail book or audit trail software can be helpful in avoiding such errors. However, such measures are typically reliant on user input, and are not readily able to verify directly that some of the steps have been correctly performed.

US 9 433 474 discloses a two-part decontamination system suitable for cleaning and disinfecting a medical instrument. The system comprises a first reagent, contained in a dispenser, and a second reagent absorbed or impregnated onto a wipe, which reacts when mixed to provide a disinfecting composition.

US 2020/043604 discloses an automated sterilization system which uses artificial intelligence for processing, automating, controlling and tracking of surgical lab instruments. The system comprises modules to facilitate auditing, reporting and context based training associated with the surgical lab instruments.

CN 110 263 689 discloses a hand washing monitoring method and an associated hand washing device. The method encompasses using artificial intelligence to compare a standard hand washing gesture with a real time video of hand washing with a view to reducing the risk of disease transmission during hand washing.

Against that background, it would be desirable to provide solutions for validation of procedures during decontamination processes that are less reliant on user input and that are able directly to validate more of the steps in a decontamination process.

### SUMMARY OF THE INVENTION

From a first aspect, the present invention resides in a method for validating a decontamination procedure performed using a decontamination system that comprises a first product for use in a first step of the procedure and a second product for use in a subsequent second step of the procedure. The method comprises capturing a video stream of a work area in which the decontamination procedure is carried out by a user, analysing the video stream to identify when the first product is present in the work area and when the second product is present in the work area, determining, based on said identification, that the first and second products have been presented in the correct order in the decontamination procedure, and causing a corresponding indication to be provided to the user.

With this method, validation that the correct sequence of steps has been performed in use of a multi-stage decontamination system can be based on the automated assessment of a user's actions through analysis of the video stream, which is of substantial benefit in ensuring that the user completes the procedure correctly. The method provides the user with a positive indication that the stages have been performed in the correct sequence, thereby ensuring that the decontamination process is optimally effective and improving user confidence.

Analysing the video stream may comprise using a trained neural network to identify when the first and second products are present in the work area. With suitable training, the neural network is able to reliably and rapidly identify the first product and the second product.

Analysing the video stream may comprise detecting a first product container containing the first product and/or a second product container containing the second product. To enable the product containers to be distinguished from one another, the first product container and the second product container may be differentiated in appearance by different indicia (such as labels, logos or designs). Alternatively, or in addition, the first product container and the second product container may be differentiated in appearance by different colours. For example, the containers may be substantially of single, different colours, or the indicia on each container may be of different colours.

Analysing the video stream may further comprise determining, from the video stream, information relating to the first product and/or information relating to the second product. In one implementation, the determined information relating to the first product and/or the determined information relating to the second product is retrieved from a database that cross-references product information and product container appearance. Alternatively, the determined information may be obtained by reading machine-readable information, such as a barcode or matrix code.

The method may include checking, using the determined information relating to the first product and/or the determined information relating to the second product, whether the first product and/or the second product are approved for use in the decontamination system, and providing a corresponding indication to the user. Similarly, the method may include checking said determined information relating to the first product and/or said determined information relating to the second product against corresponding user-input information, and providing a corresponding indication to the user.

The decontamination procedure may be for a medical device, in which case the method preferably comprises checking compatibility between the medical device and the decontamination system using said determined information relating to the first product and/or said determined information relating to the second product. To this end, the method may comprise analysing the video stream to determine information relating to the medical device, and checking compatibility between the medical device and the decontamination system using said determined information relating to the medical device.

The method may also provide a means for electronically logging information concerning the decontamination procedure. For example, the method may comprise electronically logging the determined information relating to the first product and/or the determined information relating to the second product, and/or electronically logging that the first and second products have been presented in the correct order in the decontamination procedure.

The first step may be immediately followed by the second step in the decontamination process. However, the second step need not follow consecutively from the first step, and one or more intermediate steps may be provided.

The decontamination system may comprise a third product for use in a third step of the procedure subsequent to the second step, in which case the method may comprise analysing the video stream to identify when the third product is present in the work area, and determining that the first, second and third products have been presented in the correct order in the decontamination procedure.

In a preferred embodiment, the first product comprises a pre-clean composition and the second product comprises a disinfecting composition. The third product, when present, preferably comprises a rinse composition.

In addition to providing a positive indication that the steps have been performed in the correct order, it is also possible for the method to provide an indication that a possible error has occurred, allowing the user to take corrective action. To that end, the method may further comprise, when analysing the video stream, identifying one or more alert events in the video stream that correspond with potential errors being made during performance of the decontamination procedure, and upon identification of an alert event, causing a corresponding alert to be provided to the user. The alert event or one of the alert events may comprise an incorrect product being present in the work area. In another example, the alert event or one of the alert events comprises the first product, the second product and, when used, the third product being presented in an incorrect order in the work area.

The invention also extends, in a second aspect, to an apparatus for validating a decontamination procedure performed using a decontamination system, the decontamination system comprising a first product for use in a first step of the procedure and a second product for use in a subsequent second step of the procedure, the apparatus comprising a camera system for capturing a video stream of a work area in which the decontamination procedure is carried out by a user, an output device for providing audio, textual and/or visual indications to the user, an image analysis module configured to receive the video stream and to identify that the first product is present in the work area and that the second product is present in the work area, and a validator module configured to determine, based on said identification, if the first and second products have been presented in the correct order in the decontamination procedure, and to cause the output device to provide a corresponding indication to the user. Preferably, the image analysis module comprises a neural network-based classifier trained to recognise said first and second products. The apparatus may further comprise a logging module configured to electronically log the first and second products have been presented in the correct order in the decontamination procedure. Conveniently, the apparatus may comprise a smartphone or tablet computer device.

The use of an apparatus according to the second aspect to perform the method of the first aspect is also disclosed.

In a third aspect, the invention extends to a decontamination system for performing a decontamination procedure, comprising a first product for use in a first step of the procedure, a second product for use in a subsequent second step of the procedure; and an apparatus according to the second aspect of the invention for validating the decontamination procedure in use of the decontamination system. The first product of the system may be provided in a first product container and the second product may be provided in a second product container, with the first and second product containers being differentiated in appearance. In this case, the image analysis module is preferably configured to detect the presence in the work area of the first product container and the second product container.

The present invention can be used to validate the sequence of steps performed in the use of substantially any decontamination system that involves the successive use of different products, including systems in which a mixing procedure is required to produce an active disinfecting or cleaning composition. In those cases, embodiments of the present invention may be extended to verify that the mixing procedure has been correctly performed. The decontamination process may be used to decontaminate a medical instrument or device, in which case embodiments of the invention may validate the correct cleaning procedure for the device in addition to the sequence of steps.

In the context of this specification, the term "validating a procedure" is used to refer to confirming by suitable means that one or more particular steps have been taken or avoided, and/or that one or more specific products have been used, when carrying out the procedure in question. The procedure that is validated need not encompass an entire process for decontaminating a device, object or surface, but may instead be only a part of a longer decontamination process.

The term "decontamination process" is used to refer to a sequence of steps by which an object or surface is cleaned, prepared for cleaning, disinfected, sterilised, rinsed and so on, including steps for the preparation of materials and products for use in such activities. The term "decontamination procedure" is used to refer to a single such step within a decontamination process, a combination of such steps as a subset of a longer decontamination process, or all of the steps of a decontamination process, as the context demands.

### BRIEF DESCRIPTION OF THE DRAWINGS

Embodiments of the present invention will now be described, by way of example only, with reference to the accompanying drawings, in which like reference numerals are used for like features, and in which:
Figure 1 shows a known decontamination system to which embodiments of the invention can be applied;
Figure 2 shows a sequence of steps in a decontamination process using the system of Figure 1;
Figure 3 shows an apparatus for validating a decontamination procedure; and
Figures 4 to 12 are images captured from a video stream showing events taking place during a decontamination process for a medical device.

### DESCRIPTION OF THE PREFERRED EMBODIMENTS

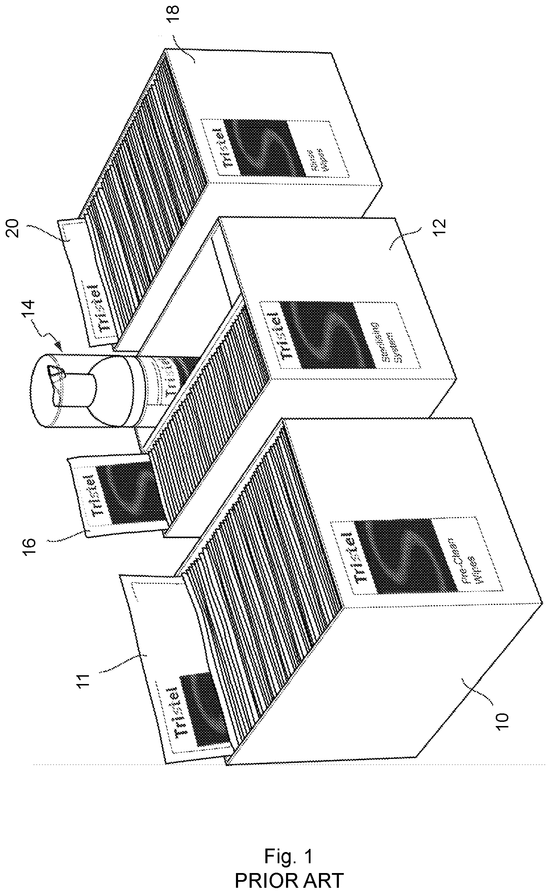

Embodiments of the present invention provide methods and apparatus for automatically or semi-automatically validating various procedures or steps that a user must undertake when performing a decontamination process. The following examples refer to the decontamination system and corresponding decontamination process described above with reference to Figures 1 and 2, but embodiments of the invention are equally applicable to other decontamination systems and processes.

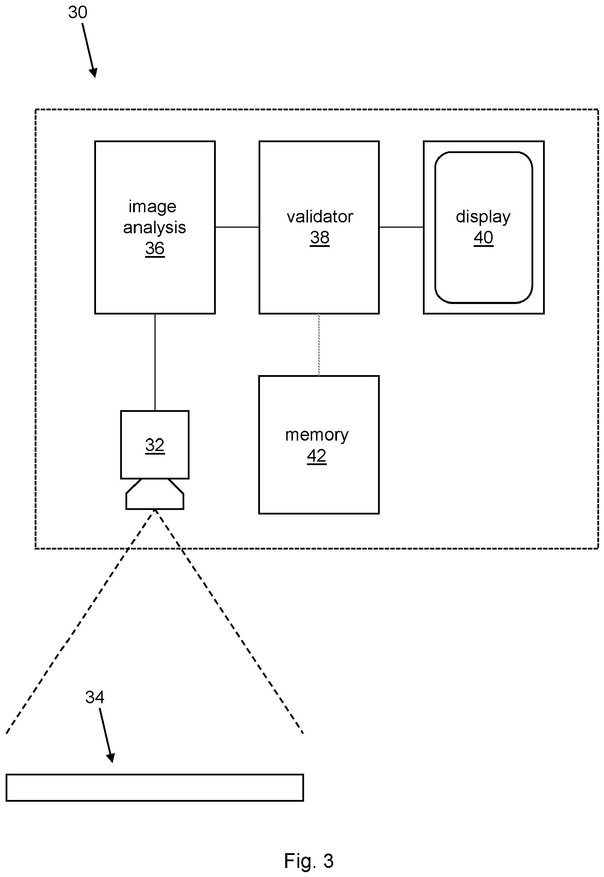

Figure 3 is a schematic illustration of an apparatus for validating a procedure. In this example, the apparatus comprises a portable device 30 having a camera system 32 for capturing a video stream of a work area 34.

The camera system 32 outputs the video stream to an image analysis module 36. As will be explained in more detail below, the image analysis module 36 analyses the video stream to identify when certain pre-defined events or actions are being performed in the work area. The image analysis module 36 outputs corresponding event data to a validator module 38.

The validator module 38 analyses the received event data to determine whether a predefined procedure has been correctly completed, or if any potential errors are being made. After making such a determination, the validator module 38 causes a display 40 to provide a corresponding indication to the user. The validator module 38 may also cause an indication to be provided via an audio output (not shown), such as a loudspeaker or headphone output.

The validator module 38 is also configured to log data in a memory 42. In this way, the validator module 38 can record, in the memory 42, that one or more predefined procedures have been correctly completed and/or that one or more potential errors have been observed in the video stream.

This logged event information may be correlated with additional information, such as a user name and other user details, patient details, a location in which the procedure is being carried out, the type of device, surface or other item being decontaminated, the serial number of a device, details of the decontamination system and its constituent parts, such as serial, batch or lot numbers, dates of manufacture, expiry dates and so on. Such additional information may be input by a user through a suitable interface (not shown), typically a touch-screen, may be captured by reading a barcode or matrix code presented to the camera, or may be determined by analysis of the video stream. In the latter case, the image analysis module 38 may for example be configured to recognise the containers (i.e. the sachets 11, 16, 20 and dispenser 14) that contain the various products used in the decontamination system, and the validator module 38 may be configured to retrieve stored product type information corresponding to each identified container and to record that information along with the logged event information.

In these ways, the memory 42 stores a partial or full audit trail of the decontamination process that can be stored in the memory 42 for subsequent retrieval, and/or transmitted to a server or another device using a suitable communication protocol or network for record-keeping purposes.

The apparatus may be provided as a self-contained, portable device 30, such as a smartphone or tablet computer. Conveniently, conventional smartphones, tablet computers and similar devices already include a camera system, data storage, a display, an audio output and communication devices for network connectivity, along with a processor that can be configured to execute suitable instructions to cause the methods described herein to be performed. The image analysis module 36 and validator module 38 are therefore preferably realised in software for execution by the processor. The software may be embodied as an installable application that runs within a device operating system environment. The image analysis module 36 and/or the validator module 38 may also be provided, in whole or in part, by cloud-based services with which the device 30 is configured to communicate.

The camera system may comprise a single camera or multiple cameras, and it is conceivable that non-visible light cameras (such as thermal or depth cameras) could be provided to feed additional data to the image analysis module 36.

The device 30 is preferably mounted on a suitable stand, with the camera apparatus 32 directed towards the work area 34, to free both of the user's hands for performing the decontamination process. However, in some cases it may also be possible for the user to hand-hold the device 30 for some or all of the process.

The image analysis module 36 can use any suitable method to analyse the video stream, but preferably a machine learning approach is used. For example, the image analysis module 36 may employ a trained artificial neural network (such as a convolutional neural network and/or a recurrent neural network) to identify, classify or characterise objects and actions that appear or occur in the work area 34 on a frame-by-frame basis or across multiple frames. Supervised learning can be readily employed by training the network using video footage and/or images of multiple versions of the events to be identified. Semi-supervised learning, active learning and user feedback input can also be used, to achieve ongoing improvements in accuracy. In embodiments, these approaches can be used to refine the neural network for increased accuracy with a particular user and/or a particular environment or work area.

Various suitable neural network models will be familiar to those skilled in the art of machine learning-based video analysis and will not be discussed in detail here. One suitable neural network architecture is based on MobileNet, which provides a convolutional neural network for extracting framewise features from the video stream. This is combined with an LTSM (long short-term memory) recurrent neural network to aggregate temporal information (i.e. movement between frames). Other possibilities include two-stream convolutional neural networks.

It is to be noted that, in each implementation of the invention, the image analysis module 36 need only be able to identify a relatively small number of different events to allow the validator module 38 to determine accurately whether the procedure in question has been correctly performed or whether potential errors have occurred.

Some of the events identified by the image analysis module 36 may correspond to a step in the decontamination procedure being correctly performed. Examples of such "decontamination events" include picking up the correct product, correctly combining and mixing the two parts of the disinfectant system, applying a decontaminating composition to a device in the correct way, and so on. Others of the events may correspond to potential errors being made by the user. Examples of such "alert events" include selecting the wrong product for a particular stage in the procedure, cleaning a device too quickly or in the wrong direction, applying the wrong quantity of one of the parts of the two-part disinfectant system to the other part, mixing the two parts incorrectly or for an insufficient time, shaking the dispenser before use, placing the device in a designated clean region of the work area before decontamination or in a designated dirty region of the work area after decontamination, failure to wear gloves and/or other protective equipment, and so on.

The neural network is trained for all of the events or tasks to be identified in a multi-task learning environment. For the characterisation of each event or task, a hierarchical representation of the outcome may be used. For example, the task of detecting the type of product contained within a sachet is conditional on observing the sachet. In this way, the outcomes are forced to be consistent.

A training dataset covering all of the events to be identified can be produced from multiple demonstration videos in which events are labelled, including multiple examples showing the same event with suitable variations to reflect the main sources of variability expected in use. Such variations may include variations in cameras, portrait or landscape orientation, camera tilt, distance between the camera and the work area, background colour and material, lighting colour, source, intensity and direction, glove colour, user's skin colour, gender, handedness and proficiency in the usage of the products, and so on. Further variability can be synthetically added by data augmentation, for example to introduce variability in brightness, different light conditions, horizontal and vertical flips to simulate camera orientation, shearing and rotation to simulate camera tilting. For subsequent testing, a testing dataset may be used in which different demonstration videos, for example with a different user and/or different settings of the above variables, are provided compared with those in the training dataset.

To facilitate learning of procedures without labels, a self-supervised learning approach can added. For example, the frame ordering in videos showing the correct procedure can be shuffled and the network can then be trained to recognise the original frame order. Other examples of self-supervised learning include image inpainting (removing a part of an image and training the network to reconstruct it, to learn the structure of the objects/medical device), and image recolouring (to make the model learn a colour transition upon mixing and/or an expected colour of a sachet or other container).

The image analysis module 36 outputs the identified decontamination events and the identified alert events to the validator module 38. The validator module 38 may determine that each step in a procedure has been correctly performed when the output of the image analysis module 36 indicates that the required procedure-specific decontamination event or events have occurred. To that end, the validator module 38 may employ a finite state machine or a hidden Markov model to convert the framewise prediction of events output from the image analysis model 36 to a global assessment of the sequence of steps performed in the procedure. When more than one decontamination event is required for the correct performance of a procedure, the validator module 38 may check that all of the required events have occurred, and that they have occurred in the correct order. The validator module 38 may also check that no alert events have occurred, or that the error corresponding to an identified alert event has been subsequently corrected (for example through the observation of a corresponding decontamination event immediately afterwards). Some alert events may correspond to immediately-correctable errors, such as picking up an incorrect product, or having the user's hand or parts of the decontamination system obscured or positioned outside the field of view, while others, such as applying the incorrect product to a device, may require the decontamination process to be re-started.

In addition to the ability to log these outcomes in the memory 42 as part of an audit trail, the device 30 advantageously can also provide immediate feedback to the user, by the display of graphical and/or textual indications on the display 40 and/or by the playing of sounds and/or speech through the audio output of the device. In this way, when each step of a specific procedure is correctly performed, an indication can be provided to the user to confirm both that the step has been completed, and that the device 30 has recorded that fact. Similarly, if a potential error is identified, giving rise to an alert event, the user can be prompted by a suitable indication to take suitable corrective action.

The device 30 can also be used to guide a user through the procedure, by providing prompts or instructions relating to the next steps to be performed using the display 40 and/or the audio output. For example, the validator module 38 may cause an instruction relating to the next step of the procedure to be displayed after a particular decontamination event has been identified. In another example, where a particular action must be performed for a specific time, the validator module 38 may cause a timer to be displayed as a guide for the user when an event indicating the start of that action is identified.

To enable the validator module 38 to operate in these ways, suitable procedure data relating to each specific procedure can be stored in the memory 42. The procedure data may include, for example, a list of required events and the order in which they must be undertaken, predefined times or other parameters for individual steps, and a list of possible alert events that correspond to potential errors. The validator module 38 can compare the identified decontamination and alert events to the stored procedure data to determine if each step in the procedure has been performed correctly or if errors have occurred, and can retrieve information about the expected next step to trigger the display of instructions or other actions such as the start of countdown timer. The memory 42 may store procedure datasets for a plurality of possible procedures, so that the validator module 38 can select whichever procedure dataset is appropriate for the procedure being performed. The procedure data may include corrections to allow for adjustment of parameters to compensate for environmental factors, such as temperature and humidity, which may be determined by the device 30 using suitable sensors and/or input by the user.

### Example 1

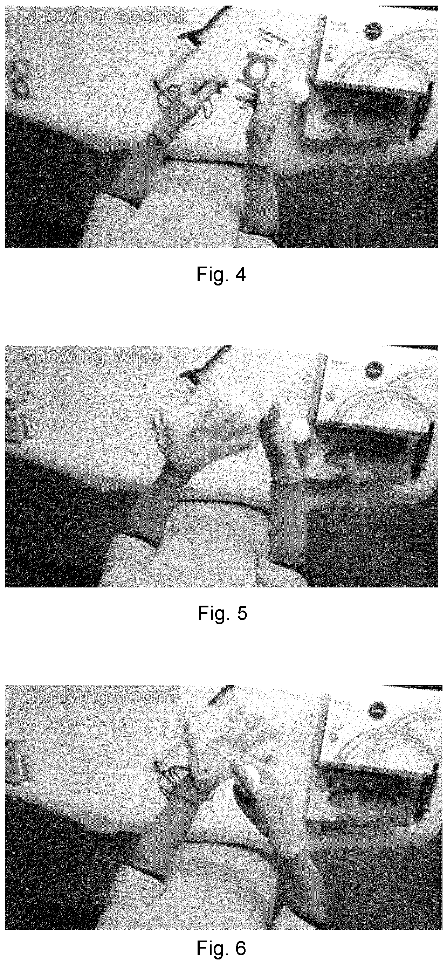

The following example describes the use of the device 30 to validate a decontamination process for a medical device in which a decontamination system of the type shown in Figure 1 is used. The medical device may for example be an endoscope, nasendoscope, transvaginal probe or similar device, with a proximal handle part and a distal invasive end part. Figures 4 to 12 show example frames captured by the camera system 32, which in this case is positioned above the work area. As an aid to understanding, the output provided by the image analysis module 36 is indicated in the top left corner of each image.

Step 1. First, the type of medical device to be decontaminated is identified and logged. This may be done automatically by image analysis of the video stream when the device is placed in the work area, in which case the user is subsequently prompted to confirm that the medical device type has been correctly identified. Alternatively, the medical device type may be entered by the user, for example by selection from a list of predefined options.

Step 2. The unique serial number of the device is captured and logged. Again, this may be done by image analysis, for example by capturing a barcode or matrix (QR) code or by reading text directly; alternatively, the serial number may be entered by the user. Once captured, the serial number is checked to ensure that it is possible for that type of device.

Step 3. The device 30 displays a prompt to the user to prepare a pre-cleaning wipe.

Step 4. The device 30 identifies and acknowledges that an appropriate pre-cleaning product is to be used. The image analysis module 36 identifies, as a decontamination event, the presence in the work area of an approved pre-clean wipe sachet as it is presented to the camera by the user. The pre-clean wipe sachets are differentiated from the sachets used later in the process by differently-coloured indicia, so that the image analysis module 36 can distinguish between the various sachets. If a sachet is presented that does not correspond to the expected pre-clean wipe sachet, an alert event is identified and the device 30 indicates that the wrong sachet has been selected. The identified sachet type may also be checked against a stored stock list of suitable pre-cleaning products or user-inputted systems. The presence of the pre-cleaning product, optionally along with the type of the product and further information such as a batch number or expiry date, is logged.

Step 5. Image analysis is used to identify the proximal handle part of the device and the distal invasive end part of the device.

Step 6. The device 30 displays a prompt to the user to commence pre-cleaning of the device, and then monitors the video stream for possible alert events. For example, if pre-cleaning starts from the dirtier invasive end part instead of the cleaner proximal handle part, or if the wipe is moved in the wrong direction, an error will be indicated. Similarly, if the pre-cleaning action performed by the user is too fast or conflicts with pre-determined product specific instructions for use data, an error will be indicated.

Step 7. When the image analysis module 36 identifies that the device has been precleaned from the clean part to the dirty part in accordance with product specific user instructions without alert events being identified, the device 30 provides an acknowledgement to the user.

Step 8. The device 30 prompts the user to prepare a disinfectant wipe.

Step 9. The device 30 identifies and acknowledges that an appropriate disinfectant product is to be used. As in step 4 above, the image analysis module 36 identifies, as a decontamination event, the presence in the work area of an approved disinfectant wipe sachet as it is presented to the camera by the user. This is shown in Figure 4. If a sachet is presented that does not correspond to the expected disinfectant wipe sachet, an alert event is identified and the device 30 indicates that the wrong sachet has been selected. The identified sachet type may also be checked against a stored stock list of suitable disinfectant products or user-inputted systems. An additional verification check cross-references the disinfectant product against the previously used pre-clean product to ensure they are approved for use together. The presence of the disinfectant wipe, optionally along with the type of the product and further information such as a batch number or expiry date, is logged.

Step 9A. The device 30 identifies and acknowledges that an appropriate disinfectant activator product is to be used. Here, the image analysis module 36 identifies, as a decontamination event, the presence in the work area of an approved dispenser of the activator foam as it is presented to the camera by the user. The same checks may be applied for the activator product in this step as in step 9 above.

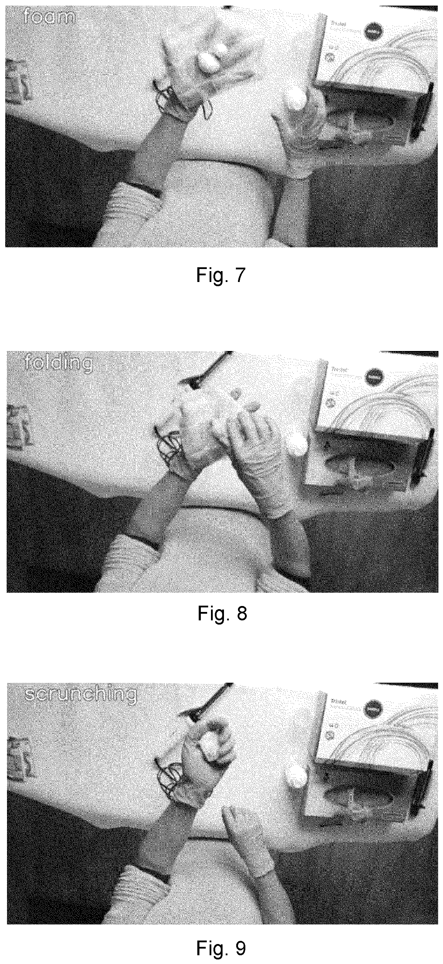

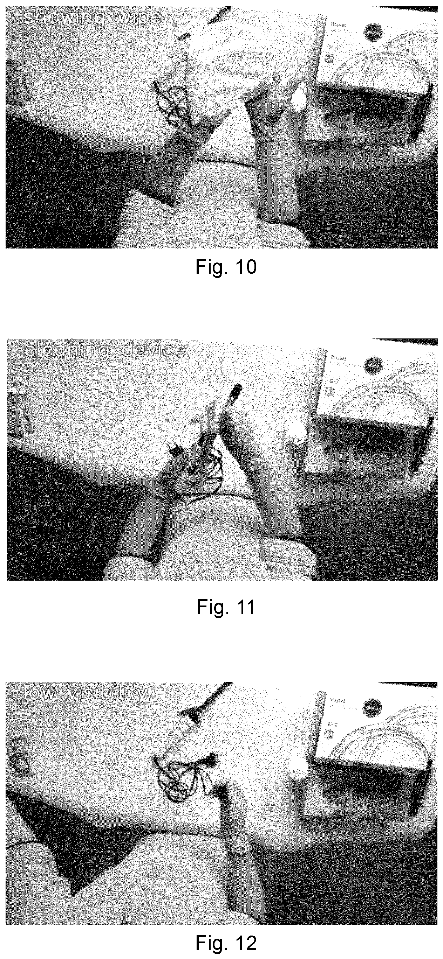

Step 9B. The device 30 prompts the user to apply the activator foam to the wipe, and then monitors the video stream for the following events:
(a) A disinfectant wipe has been removed from the sachet and is visible in the work area (see Figure 5).
(b) Two aliquots of foam have been added to the wipe (two aliquots corresponding to the correct quantity to be added to a single wipe, in this example). Figure 6 shows the identification of the step of dispensing foam onto the wipe from the dispenser, and Figure 7 shows the aliquots of foam on the wipe.
(c) The wipe has been folded (Figure 8) and scrunched (Figure 9) to distribute the foam throughout the wipe for at least a minimum period of time to ensure full activation of the disinfecting composition.
(d) The wipe has been unfolded, has a uniform appearance, and is ready for use (Figure 10).

After each event has been identified, the device 30 logs the event, displays an acknowledgement that the corresponding step has been correctly performed, and prompts the user to perform the next step. If any errors are made, such as fewer or more aliquots of foam being dispensed than are required, an alert is displayed to the user. Optionally, the size of each aliquot is checked to ensure that the pump has been correctly operated each time.

Step 10. Image analysis is again used to identify the proximal handle part of the device and the distal invasive end part of the device.

Step 11. The device 30 displays a prompt to the user to commence disinfection of the device, and then monitors the video stream for possible alert events. Figure 11 shows identification of the disinfecting wipe being applied to the device. If disinfection starts from the invasive end part instead of the cleaner proximal handle part, or if the wipe is moved in the wrong direction, an error will be indicated. Similarly, if the disinfection action performed by the user is too fast or conflicts with pre-determined product specific instructions for use data will signal, an error will be indicated.

Step 12. The image analysis module 36 identifies when the disinfecting wipe has been wiped over all of the medical device. The device 30 then displays a countdown timer and starts a countdown for the contact time of the disinfection process. If, during the wiping process, wiping is stopped or interrupted or otherwise not conducted at a uniform speed along the length of the device, or if the wipe is removed from the device, an error will be indicated. Similarly, if the user touches the device or another item (such as a wipe) contacts the device during the countdown, an error will be indicated.

Step 13. When the image analysis module 36 identifies that the device has been disinfected from the clean part to the dirty part in accordance with product specific user instructions, and that the required contact time has been observed, without alert events being identified, the device 30 provides an acknowledgement to the user.

Step 14. If the products used in the precleaning and disinfection stage do not require final rinsing the device 30 will indicate that the decontamination process is complete. If the predefined parameters indicate that the product set used requires rinsing the device 30 will prompt to proceed to step 15.

Step 15. If rinsing is required, the device 30 will prompt the user to prepare a rinse wipe.

Step 16. The device 30 identifies and acknowledges that an appropriate rinse wipe product is to be used. The image analysis module 36 identifies, as a decontamination event, the presence in the work area of an approved rinse wipe sachet as it is presented to the camera by the user. If a sachet is presented that does not correspond to the expected rinse wipe sachet, an alert event is identified and the device 30 indicates that the wrong sachet has been selected. The identified sachet type may also be checked against a stored stock list of suitable rinse products or user-inputted systems, and may be checked for compatibility with the pre-clean and disinfectant wipes used in the previous steps. The presence of the rinse product, optionally along with the type of the product and further information such as a batch number or expiry date, is logged.

Step 17. Once again, image analysis is used to identify the proximal handle part of the device and the distal invasive end part of the device.

Step 18. The device 30 displays a prompt to the user to commence rinsing of the device, and then monitors the video stream for possible alert events. For example, if rinsing starts from the invasive end part instead of the cleaner proximal handle part, or if the wipe is moved in the wrong direction, an error will be indicated.

Step 19. When the image analysis module 36 identifies that the device has been rinsed from the clean part to the dirty part in accordance with product specific user instructions without alert events being identified, the device 30 provides an acknowledgement to the user.

Step 20. At the end of the process, the device 30 provides a successful process validation code (if required by the user) and logs the details of the process in the memory 42. It is also possible for the device 30 to log the recommended storage time before re-disinfection is required.

The image analysis operations in the above steps rely upon the procedures being carried out within the field of view of the camera system. Accordingly, throughout all of the steps, the device 30 will provide an error indication to the user if the user's hands are not visible in the video stream because they are obscured or outside the visible area, as shown in Figure 12.

In the above steps, an "approved" product means that that the product is intended for use for the intended application, approved by the user's establishment for the intended application and/or approved by the medical device manufacturer.

### Example 2

In this example, the two-part disinfectant system 12 includes, in one or both of the parts, a component that exhibits a colour change when the two parts are mixed. In this case, the colour change can be identified in the video stream to verify the complete mixing of the two parts of the disinfectant system 12, in addition to the verification of the physical mixing process described in step 9B of Example 1.

Accordingly, the verification steps associated with the correct preparation of an activated disinfecting wipe in this example are as follows:
Step 1. Identifying a disinfecting wipe sachet, checking compatibility, and acknowledging to the user.
Step 2. Identifying an activator foam dispenser, checking compatibility, and acknowledging to the user.
Step 3. Identifying a non-activated wipe when removed from the sachet. At this stage, an error is indicated if the wipe is not of the correct starting colour.
Step 4. Identifying the dispensing of the foam to the wipe and, optionally, the correct number of aliquots of foam.
Step 5. Identifying the folding and scrunching of the wipe for the appropriate time.
Step 6. Identifying that, after scrunching, the disinfecting wipe is of the correct post-change colour, and that the colour is uniform across the wipe.

It is also possible that the colour change alone could be used to verify complete mixing of the two parts of the disinfectant system, in which case steps 4 and 5 could be omitted.

It will be understood that not all of the steps set out in the examples above need to be identified as events by the image analysis module 36 and taken into account by the validation module 38 in order for the device 30 to reliably validate the respective processes. In embodiments of the invention, recognition of some of the steps can be omitted. Similarly, additional steps not discussed in these examples may be recognised and used in the validation method.

The above examples are merely illustrative of decontamination processes that can be validated by the present invention. Many variations are possible.

For example, WO 2006/079822 A1 describes a disinfecting system in which the first and second reagents are each carried in aqueous media to which foam promoters are added, so that both the first and second parts of the system are dispensed as first and second foams respectively. The first and second foams are mixed to generate the disinfecting composition, which may then be applied to an item or surface to be disinfected directly or with a wipe. The first and second foams may be dispensed separately and manually mixed together, or may be dispensed simultaneously from a dispenser that pre-mixes the foams during dispensing. In such a system, the verification process could include identifying the or each foam dispenser, identifying the dispensing of suitable quantities of each foam (or, when applicable, the pre-mixed foam), identifying that a manual mixing process has been performed (such as stirring the foams for a predefined period, or scrunching them together on a wipe), identifying that the mixed foams have been allowed to dwell for a predefined period before use, and/or applying the foams separately or in a mixed form to a wipe or other substrate. As in Example 2 above, one or both of the parts may include a component that exhibits a colour change when the two parts are mixed, in which case the verification process may comprise identifying that, after mixing, the mixed foam is of the correct post-change colour, and that the colour is uniform within the foam.

As already indicated, the process of Example 1 may omit the rinse stage if it is not necessary in a particular application. In this case, only the pre-clean and disinfection stages would be used. It is also conceivable that only the disinfection stage and the rinse stage could be used, with the omission of the pre-clean stage. When at least two stages are used, using two different products, the validation process preferably includes the steps of identifying each product being used and verifying that they are being used in the correct order.

Although not part of the present invention, it is also possible for a single-stage disinfection or decontamination process to be validated using the approach described above. For example, in some applications, the use only of a cleaning or disinfecting wipe may be necessary to provide the required degree of decontamination. In such a case, the validation process may focus primarily on determining that the wipe has been correctly applied to a medical device or other instrument (for example as described in steps 10 to 13 of Example 1). It will be appreciated that, in some cases, the correct application of the wipe may differ from that described above. For instance, it may not be necessary to wipe from the proximal handle part towards the operative distal part, and in such cases the validation process may determine for example that the wipe has been applied to the whole surface or to a specific area of a medical device.

When a two-part disinfection system is used, such as a chlorine dioxide-based disinfection system described in WO 2005/011756, validation of the steps that ensure correct mixing of the two parts of the disinfection system is useful. However, a single-part disinfecting composition might be appropriate for some applications, and in these cases validation of other procedural steps, such as the correct selection of products and the correct cleaning of a device, is possible. Single-part disinfecting compositions may include, but are not limited to, hydrogen peroxide, peracetic acids, sodium hypochlorite, peroxy acids, quaternary ammonium compounds, and so on.

The above-described methods and apparatus are not limited to the validation of decontamination processes that use wipes. Substantially any type of product, such as foams, liquids, spray compositions and powders could be used in substantially any combination in a decontamination process and validated using the above-described methods and apparatus. Validation based on the identification of product containers, for example, would still be possible. It would also be possible to train the image analysis module to identify and verify actions such as spraying a medical device with a foam or liquid product or dipping a medical device in a container of liquid.

While the above examples refer to decontamination of a medical device, the validation approach described here can also be applied to decontamination of surfaces or items that cannot readily be placed in the field of view of the camera system. In such cases, validation can for example be performed for the selection of the correct product or sequence of products, and/or the correct mixing of a two-part disinfection system.

Further modifications and variations not explicitly described above may also be contemplated without departing from the scope of the invention as defined in the appended claims.

## Claims

1. A method for validating a decontamination procedure performed using a decontamination system, the decontamination system comprising a first product for use in a first step of the procedure and a second product for use in a subsequent second step of the procedure, the method comprising:
capturing a video stream of a work area (34) in which the decontamination procedure is carried out by a user;
analysing the video stream to identify when the first product is present in the work area (34) and when the second product is present in the work area (34);
determining, based on said identification, that the first and second products have been presented in the correct order in the decontamination procedure; and
causing a corresponding indication to be provided to the user.

2. A method according to Claim 1, wherein analysing the video stream comprises using a trained neural network to identify when the first and second products are present in the work area (34).

3. A method according to Claim 1 or Claim 2, wherein analysing the video stream comprises detecting a first product container containing the first product and/or a second product container containing the second product.

4. A method according to Claim 3, wherein the first product container and the second product container are differentiated in appearance by different indicia; and/or by different colours.

5. A method according to any preceding claim, wherein analysing the video stream further comprises determining, from the video stream, information relating to the first product and/or information relating to the second product, and optionally, electronically logging said determined information relating to the first product and/or said determined information relating to the second product.

6. A method according to Claim 5 when dependent on Claim 3 or Claim 4, wherein said determined information relating to the first product and/or said determined information relating to the second product is retrieved from a database that cross-references product information and product container appearance.

7. A method according to Claim 5 or Claim 6, comprising checking, using said determined information relating to the first product and/or said determined information relating to the second product, whether the first product and/or the second product are approved for use in the decontamination system, and providing a corresponding indication to the user.

8. A method according to any of Claims 5 to 7, comprising checking said determined information relating to the first product and/or said determined information relating to the second product against corresponding user-input information, and providing a corresponding indication to the user.

9. A method according to any of Claims 5 to 8, wherein the decontamination procedure is for a medical device, and wherein the method comprises checking compatibility between the medical device and the decontamination system using said determined information relating to the first product and/or said determined information relating to the second product; and wherein the method optionally comprises analysing the video stream to determine information relating to the medical device, and checking compatibility between the medical device and the decontamination system using said determined information relating to the medical device.

10. A method according to any preceding claim, wherein the first product comprises a pre-clean composition and the second product comprises a disinfecting composition.

11. A method according to any preceding claim, further comprising:
when analysing the video stream, identifying one or more alert events in the video stream that correspond with potential errors being made during performance of the decontamination procedure;
and upon identification of an alert event, causing a corresponding alert to be provided to the user;
wherein the alert event or one of the alert events optionally comprises:
- an incorrect product being present in the work area (34); or
- the first product and the second product being presented in an incorrect order in the work area (34).

12. A method according to any preceding claim, further comprising electronically logging that the first and second products have been presented in the correct order in the decontamination procedure.

13. Apparatus for validating a decontamination procedure performed using a decontamination system, the decontamination system comprising a first product for use in a first step of the procedure and a second product for use in a subsequent second step of the procedure, the apparatus comprising:
a camera system for capturing a video stream of a work area (34) in which the decontamination procedure is carried out by a user;
an output device for providing audio, textual and/or visual indications to the user;
an image analysis module (36) configured to receive the video stream and to identify that the first product is present in the work area (34) and that the second product is present in the work area (34), the image analysis module (36) optionally comprising a neural network-based classifier trained to recognise said first and second products;
a validator module (38) configured to determine, based on said identification, if the first and second products have been presented in the correct order in the decontamination procedure, and to cause the output device to provide a corresponding indication to the user;
and, optionally, a logging module configured to electronically log the first and second products have been presented in the correct order in the decontamination procedure.

14. A decontamination system for performing a decontamination procedure, comprising:
a first product for use in a first step of the procedure;
a second product for use in a subsequent second step of the procedure; and
an apparatus according to Claim 13 for validating the decontamination procedure in use of the decontamination system.

15. A decontamination system according to Claim 14, wherein the first product is provided in a first product container and the second product is provided in a second product container, the first and second product containers being differentiated in appearance, and wherein the image analysis module (36) is configured to detect the presence in the work area (34) of the first product container and the second product container.

## Patentansprüche

1. Verfahren zum Validieren eines
Dekontaminationsverfahrens, welches unter Verwendung eines Dekontaminationssystems durchgeführt wird, wobei das Dekontaminationssystem ein erstes Erzeugnis zur Verwendung in einem ersten Schritt des Verfahrens und ein zweites Erzeugnis zur Verwendung in einem nachfolgenden zweiten Schritt des Verfahrens umfasst, wobei das Verfahren folgendes umfasst:
Aufnehmen eines Videodatenstroms eines Arbeitsbereichs (34), in welchem das Dekontaminationsverfahren durch einen Benutzer ausgeführt wird;
Analysieren des Videodatenstroms, um zu identifizieren, wann das erste Erzeugnis in dem Arbeitsbereich (34) vorhanden ist und wann das zweite Erzeugnis in dem Arbeitsbereich (34) vorhanden ist;
auf der Grundlage der genannten Identifikation wird festgestellt, dass das erste und das zweite Erzeugnis in der richtigen Reihenfolge in dem Dekontaminationsverfahren eingesetzt worden sind; und
es wird bewirkt, dass ein entsprechender Hinweis für den Benutzer bereitgestellt wird.

2. Verfahren nach Anspruch 1,
bei welchem das Analysieren des Videodatenstroms umfasst, dass ein trainiertes neuronales Netzwerk verwendet wird, um zu identifizieren, wann das erste und das zweite Erzeugnis in dem Arbeitsbereich (34) vorhanden sind.

3. Verfahren nach Anspruch 1 oder Anspruch 2,
bei welchem das Analysieren des Videodatenstroms umfasst, dass ein erster Erzeugnisbehälter, der das erste Erzeugnis enthält, und/oder ein zweiter Erzeugnisbehälter, der das zweite Erzeugnis enthält, detektiert wird.

4. Verfahren nach Anspruch 3,
bei welchem der erste Erzeugnisbehälter und der zweite Erzeugnisbehälter in der äußeren Erscheinung voneinander durch unterschiedliche Indikatoren und/oder durch unterschiedliche Farben unterschieden werden.

5. Verfahren nach einem der vorigen Ansprüche,
bei welchem das Analysieren des Videodatenstroms weiter umfasst, dass aus dem Videodatenstrom Informationen, die sich auf das erste Erzeugnis beziehen, und/oder Informationen, die sich auf das zweite Erzeugnis beziehen, bestimmt werden und dass optional die bestimmten Informationen, die sich auf das erste Erzeugnis beziehen, und/oder die bestimmten Informationen, die sich auf das zweite Erzeugnis beziehen, elektronisch aufgezeichnet werden.

6. Verfahren nach Anspruch 5, sofern von Anspruch 3 oder Anspruch 4 abhängig,
bei welchem die bestimmten Informationen, die sich auf das erste Erzeugnis beziehen, und/oder die bestimmten Informationen, die sich auf das zweite Erzeugnis beziehen, aus einer Datenbank geholt werden, welche Erzeug-nisinformationen und das äußere Erscheinungsbild von Erzeugnisbehältern miteinander in Beziehung setzt.

7. Verfahren nach Anspruch 5 oder Anspruch 6,
welches umfasst, dass unter Verwendung der bestimmten Informationen, die sich auf das erste Erzeugnis beziehen, und/oder der bestimmten Informationen, die sich auf das zweite Erzeugnis beziehen, geprüft wird, ob das erste Erzeugnis und/oder das zweite Erzeugnis zur Verwendung in dem Dekontaminationssystem freigegeben sind, und dass ein entsprechender Hinweis an den Benutzer bereitgestellt wird.

8. Verfahren nach einem der Ansprüche 5-7,
welches umfasst, dass die bestimmten Informationen, die sich auf das erste Erzeugnis beziehen, und/oder die bestimmten Informationen, die sich auf das zweite Erzeugnis, entsprechend den vom Benutzer eingegebenen Informationen überprüft werden und dass ein entsprechender Hinweis an den Benutzer bereitgestellt wird.

9. Verfahren nach einem der Ansprüche 5-8,
bei welchem das Dekontaminationsverfahren für ein Medizinprodukt dient, und wobei das Verfahren umfasst, dass die Kompatibilität zwischen dem Medizinprodukt und dem Dekontaminationssystem unter Verwendung der bestimmten Informationen, die sich auf das erste Erzeugnis beziehen, und/oder der Informationen, die sie auf das zweite Erzeugnis beziehen, überprüft wird, und wobei das Verfahren optional umfasst, dass der Videodatenstrom ana-lysiert wird, um Informationen zu bestimmen, die sich auf das Medizinprodukt beziehen und dass die Kompatibilität zwischen dem Medizinprodukt und dem Dekontaminationssystem unter Verwendung der bestimmten Informationen, die sich auf das Medizinprodukt beziehen, überprüft wird.

10. Verfahren nach einem der vorigen Ansprüche,
bei welchem das erste Erzeugnis eine Vorreinigungskomposition und das zweite Erzeugnis eine Desinfektionskomposition umfasst.

11. Verfahren nach einem der vorigen Ansprüche, welches weiter folgendes umfasst:
beim Analysieren des Videodatenstroms werden ein oder eine Mehrzahl Alarmereignisse in dem Videodatenstrom identifiziert, welche mit potentiellen Fehlern korrespondieren, die während der Durchführung des Dekontaminationsverfahrens gemacht werden;
und bei Identifikation eines Alarmereignisses wird bewirkt, dass ein entsprechender Alarm für den Benutzer bereitgestellt wird;
wobei das Alarmereignis oder eines der Alarmereignisse optional folgendes umfasst:
- ein falsches Erzeugnis ist in dem Arbeitsbereich (34) vorhanden; oder
- das erste Erzeugnis und das zweite Erzeugnis sind in der falschen Reihenfolge in dem Arbeitsbereich (34) bereitgestellt worden.

12. Verfahren nach einem der vorigen Ansprüche,
welches weiter umfasst, dass elektronisch aufgezeichnet wird, dass das erste und das zweite Erzeugnis in der richtigen Reihenfolge in dem Dekontaminationsverfahren bereitgestellt worden sind.

13. Vorrichtung zur Validierung eines
Dekontaminationsverfahren, welches unter Verwendung eines Dekontaminationssystems durchgeführt wird, wobei das Dekontaminationssystem ein erstes Erzeugnis zur Verwendung in einem ersten Schritt des Verfahrens und ein zweites Erzeugnis zur Verwendung in einem nachfolgenden zweiten Schritt des Verfahrens umfasst, wobei die Vorrichtung folgendes umfasst:
ein Kamerasystem zum Aufzeichnen eines Videodatenstroms eines Arbeitsbereichs (34), in welchem das Dekontaminationsverfahren durch einen Benutzer durchgeführt wird;
eine Ausgabevorrichtung zur Bereitstellung von akustischen, textlichen und/oder visuellen Hinweisen an den Benutzer;
ein Bildanalysemodul (36), welches dazu eingerichtet ist, den Videodatenstrom zu empfangen und zu identifizieren, dass das erste Erzeugnis in dem Arbeitsbereich (34) vorhanden ist und dass das zweite Erzeugnis in dem Arbeitsbereich (34) vorhanden ist, wobei das Bildanalysemodul (36) optional einen Klassifikator auf der Grundlage eines neuronalen Netzwerkes umfasst, der dazu trainiert ist, das erste und das zweite Erzeugnis zu erkennen;
ein Validierungsmodul (38), welches dazu eingerichtet ist, auf der Grundlage der genannten Identifikation zu bestimmen, ob das erste und das zweite Erzeugnis in der richtigen Reihenfolge im Dekontaminationsverfahren bereitgestellt worden sind, und zu bewirken, dass die Ausgabeeinrichtung einen entsprechenden Hinweis an den Benutzer bereitstellt;
und optional ein Aufzeichnungsmodul, welches dazu eingerichtet ist, elektronisch aufzuzeichnen, dass das erste und das zweite Erzeugnis in der richtigen Reihenfolge im Dekontaminationsverfahren bereitgestellt worden sind.

14. Dekontaminationssystem zur Durchführung eines Dekontaminationsverfahrens, umfassend:
ein erstes Erzeugnis zur Verwendung in einem ersten Schritt des Verfahrens;
ein zweites Erzeugnis zur Verwendung in einem nachfolgenden zweiten Schritt des Verfahrens; und
eine Vorrichtung nach Anspruch 13 zum Validieren des Dekontaminationsverfahrens bei der Verwendung des Dekontaminationssystems.

15. Dekontaminationssystem nach Anspruch 14,
bei welchem das erste Erzeugnis in einem ersten Erzeugnisbehälter und das zweite Erzeugnis in einem zweiten Erzeugnisbehälter bereitgestellt sind, wobei der erste und der zweite Erzeugnisbehälter hinsichtlich ihres äußeren Erscheinungsbildes differenziert werden, und wobei das Bildanalysemodul (36) dazu eingerichtet ist, die Anwesenheit des ersten Erzeugnisbehälters und des zweiten Erzeugnisbehälters im Arbeitsbereich (34) zu detektieren.

## Revendications

1. - Procédé de validation d'une procédure de décontamination exécutée à l'aide d'un système de décontamination, le système de décontamination comprenant un premier produit destiné à être utilisé dans une première étape de la procédure et un second produit destiné à être utilisé dans une seconde étape ultérieure de la procédure, le procédé comprenant :
capturer un flux vidéo d'une zone de travail (34) dans laquelle la procédure de décontamination est réalisée par un utilisateur ;
analyser le flux vidéo pour identifier lorsque le premier produit est présent dans la zone de travail (34) et lorsque le second produit est présent dans la zone de travail (34) ;
déterminer, sur la base de ladite identification, que les premier et second produits ont été présentés dans le bon ordre dans la procédure de décontamination ; et
occasionner la fourniture d'une indication correspondante à l'utilisateur.

2. - Procédé selon la revendication 1, dans lequel l'analyse du flux vidéo comprend l'utilisation d'un réseau neuronal entraîné pour identifier lorsque les premier et second produits sont présents dans la zone de travail (34).

3. - Procédé selon la revendication 1 ou la revendication 2, dans lequel l'analyse du flux vidéo comprend la détection d'un premier récipient de produit contenant le premier produit et/ou d'un second récipient de produit contenant le second produit.

4. - Procédé selon la revendication 3, dans lequel le premier récipient de produit et le second récipient de produit sont différenciés dans leur aspect par des indices différents et/ou par des couleurs différentes.

5. - Procédé selon l'une quelconque des revendications précédentes, dans lequel l'analyse du flux vidéo comprend en outre la détermination, à partir du flux vidéo, d'informations se rapportant au premier produit et/ou d'informations se rapportant au second produit, et facultativement la journalisation électronique desdites informations déterminées se rapportant au premier produit et/ou desdites informations déterminées se rapportant au second produit.

6. - Procédé selon la revendication 5 lorsqu'elle dépend de la revendication 3 ou de la revendication 4, dans lequel lesdites informations déterminées se rapportant au premier produit et/ou lesdites informations déterminées se rapportant au second produit sont extraites d'une base de données qui croise des informations de produit avec un aspect de récipient de produit.

7. - Procédé selon la revendication 5 ou la revendication 6, comprenant la vérification, à l'aide desdites informations déterminées se rapportant au premier produit et/ou desdites informations déterminées se rapportant au second produit, pour savoir si le premier produit et/ou le second produit sont approuvés ou non pour une utilisation dans le système de décontamination, et la fourniture d'une indication correspondante à l'utilisateur.

8. - Procédé selon l'une quelconque des revendications 5 à 7, comprenant la vérification desdites informations déterminées se rapportant au premier produit et/ou desdites informations déterminées se rapportant au second produit contre des informations correspondantes entrées par l'utilisateur, et la fourniture d'une indication correspondante à l'utilisateur.

9. - Procédé selon l'une quelconque des revendications 5 à 8, dans lequel la procédure de décontamination est pour un dispositif médical, et dans lequel le procédé comprend la vérification de la compatibilité entre le dispositif médical et le système de décontamination à l'aide desdites informations déterminées se rapportant au premier produit et/ou desdites informations déterminées se rapportant au second produit ; et dans lequel le procédé comprend facultativement l'analyse du flux vidéo pour déterminer des informations se rapportant au dispositif médical, et la vérification de la compatibilité entre le dispositif médical et le système de décontamination à l'aide desdites informations déterminées se rapportant au dispositif médical.

10. - Procédé selon l'une quelconque des revendications précédentes, dans lequel le premier produit comprend une composition de pré-nettoyage et le second produit comprend une composition de désinfection.

11. - Procédé selon l'une quelconque des revendications précédentes, comprenant en outre :
lors de l'analyse du flux vidéo, identifier un ou plusieurs événements d'alerte dans le flux vidéo qui correspondent à des erreurs potentielles commises pendant la réalisation de la procédure de décontamination ; et
lors de l'identification d'un événement d'alerte, occasionner la fourniture d'une alerte correspondante à l'utilisateur,
dans lequel l'événement d'alerte ou l'un des événements d'alerte comprend facultativement :
- la présence d'un produit incorrect dans la zone de travail (34) ; ou
- la préparation du premier produit et du second produit dans un ordre incorrect dans la zone de travail (34) .

12. - Procédé selon l'une quelconque des revendications précédentes, comprenant en outre la journalisation électronique de la présentation des premier et second produits dans le bon ordre dans la procédure de décontamination.

13. - Appareil de validation d'une procédure de décontamination exécutée à l'aide d'un système de décontamination, le système de décontamination comprenant un premier produit destiné à être utilisé dans une première étape de la procédure et un second produit destiné à être utilisér dans une seconde étape ultérieure de la procédure, l'appareil comprenant :
un système de caméra pour capturer un flux vidéo d'une zone de travail (34) dans laquelle la procédure de décontamination est réalisée par un utilisateur ;
un dispositif de sortie pour fournir des indications audio, textuelles et/ou visuelles à l'utilisateur ;
un module d'analyse d'image (36) configuré pour recevoir le flux vidéo et identifier que le premier produit est présent dans la zone de travail (34) et que le second produit est présent dans la zone de travail (34), le module d'analyse d'image (36) comprenant facultativement un classificateur basé sur un réseau neuronal entraîné à reconnaître lesdits premier et second produits ;
un module de validation (38) configuré pour déterminer, sur la base de ladite identification, si les premier et second produits ont été présentés dans le bon ordre dans la procédure de décontamination, et pour amener le dispositif de sortie à fournir une indication correspondante à l'utilisateur ; et
facultativement, un module de journalisation configuré pour enregistrer électroniquement la présentation des premier et second produits dans le bon ordre dans la procédure de décontamination.

14. - Système de décontamination pour réaliser une procédure de décontamination, comprenant :
un premier produit destiné à être utilisé dans une première étape de la procédure ;
un second produit destiné à être utilisé dans une seconde étape ultérieure de la procédure ; et
un appareil selon la revendication 13 pour valider la procédure de décontamination lors de l'utilisation du système de décontamination.

15. - Système de décontamination selon la revendication 14, dans lequel le premier produit est fourni dans un premier récipient de produit et le second produit est fourni dans un second récipient de produit, les premier et second récipients de produit étant différenciés dans leur aspect, et dans lequel le module d'analyse d'image (36) est configuré pour détecter la présence du premier récipient de produit et du second récipient de produit dans la zone de travail (34).
